# EUROPÄISCHE PATENTSCHRIFT

(11) **EP 2 648 665 B1**
(45) Veröffentlichungstag und Bekanntmachung des Hinweises auf die Patenterteilung: **11.10.2017**
(21) Anmeldenummer: 10790830.3
(22) Anmeldetag: 10.12.2010
(51) Int. Cl.: A61F 9/008

(54) **LASEREINRICHTUNG, INSBESONDERE FÜR DIE OPHTHALMOLOGISCHE LASERCHIRURGIE**
LASER ARRANGEMENT, IN PARTICULAR FOR OPHTHALMOLOGICAL LASER SURGERY
DISPOSITIF LASER DESTINÉ NOTAMMENT À LA CHIRURGIE LASER OPHTALMOLOGIQUE

(43) Veröffentlichungstag der Anmeldung: 16.10.2013
(73) Patentinhaber: WaveLight GmbH, 91058 Erlangen (DE)
(72) Erfinder: WÖLFEL, Mathias, 91052 Erlangen (DE); KITTELMANN, Olaf, 14109 Berlin (DE); THUERMER, Daniel, 82205 Gilching (DE)
(74) Vertreter: Katérle, Axel
(86) Internationale Anmeldenummer: PCT/EP2010/007531
(87) Internationale Veröffentlichungsnummer: WO 2012/076031

(56) Entgegenhaltungen:
- US-A1- 2002 097 378
- US-A1- 2002 193 704
- US-A1- 2007 173 794
- US-A1- 2009 118 718

## Beschreibung

Die Erfindung befasst sich mit Lasereinrichtungen und insbesondere mit dem Testen einer Scanfunktion einer Lasereinrichtung.

Lasereinrichtungen, die mit fokussierter Laserstrahlung arbeiten, um totes oder lebendes Material (z.B. humanes Augengewebe) zu bearbeiten, weisen häufig steuerbare Komponenten auf, die eine Scanfunktion ermöglichen. Mit der Scanfunktion kann der Strahlungsfokus präzise auf unterschiedliche Positionen in einer zur Strahlungsausbreitungsrichtung orthogonalen Ebene (transversales Scannen) oder/und auf unterschiedliche Positionen längs der Strahlungsausbreitungsrichtung (longitudinales Scannen) eingestellt werden. Beispiele für Bauteile, die zum Scannen von Laserstrahlung dienen können, sind schwenkbar angeordnete Spiegel, verformbare Spiegel, elektrooptische Kristalle, verschiebbar angeordnete Linsen, brechkraftvariable Linsen, usw. Wenn im Rahmen dieser Offenbarung von Scankomponenten die Rede ist, so sind damit nicht nur die auf die Laserstrahlung einwirkenden optischen Bauteile gemeint sondern die Gesamtheit der Komponenten, die zum Scannen der Laserstrahlung benötigt werden und durch elektrische Steuersignale einer elektronischen Steueranordnung beeinflussbar sind. So gehören zu den Scankomponenten im Sinne der Erfindung insbesondere auch die zur Betätigung der optischen Scanbauteile ggf. nötigen, durch die Steuersignale der Steueranordnung ansteuerbaren Steller. Solche Steller können beispielsweise Galvanometerantriebe, Piezoantriebe, motorische Antriebe, steuerbare Spannungs- oder Stromquellen usw. umfassen. Es versteht sich, dass die vorstehende Aufzählung möglicher optischer Scanbauteile und Steller rein beispielhaft ist und nicht beschränkend zu verstehen ist.

Die Erfindung schlägt eine bevorzugt für die ophthalmologische Laserchirurgie bestimmte und eingerichtete Lasereinrichtung vor, umfassend eine Laserquelle zur Bereitstellung von Laserstrahlung, steuerbare Scankomponenten zur Einstellung einer Fokusposition der Laserstrahlung, Messkomponenten zur Erfassung von Informationen, welche für eine Ist-Position des Strahlungsfokus repräsentativ sind, sowie eine die Laserquelle und die Scankomponenten steuernde Steueranordnung, welche dazu eingerichtet ist, die Durchführung eines Testbetriebslaufs mindestens eines Teils der Scankomponenten bei abgeschalteter Laserquelle nach Maßgabe eines vorgegebenen Test-Scanmusters zu bewirken, wobei das Test-Scanmuster mindestens eine Scanbahn für den Strahlungsfokus definiert und die Steueranordnung dazu eingerichtet ist, während einer Scanbewegung entlang der Scanbahn mehrmals hintereinander eine Erfassung der Ist-Fokusposition ohne Anhalten der Scanbewegung zu bewirken und in Zuordnung zu jeder erfassten Ist-Fokusposition eine Soll-Fokusposition zu ermitteln. Der Testbetriebslauf ist sozusagen ein Trockenlauf, bei dem die Scanfunktion der Lasereinrichtung überprüft werden kann, ohne dass gleichzeitig Laserstrahlung von der Lasereinrichtung abgegeben wird. Solch ein Trockenlauf ist in US2002/0193704 beschrieben. Alterungserscheinungen, ein längerer Nichtgebrauch oder/und Datenübertragungsfehler können dazu führen, dass eine von der Steueranordnung befohlene Soll-Einstellung der Scankomponenten (entsprechend einer bestimmten Soll-Position des Strahlungsfokus) nicht präzise umgesetzt wird und die tatsächlich erzielte Ist-Einstellung der Scankomponenten von der Soll-Einstellung abweicht. Bei eingeschalteter Laserquelle würde dann die Ist-Position des Strahlungsfokus von der Soll-Position abweichen. Bei der Bearbeitung von toter Materie mag dies insofern noch hinnehmbar sein, als die Bearbeitung mit einem neuen Werkstück wiederholt werden kann, wenn sich herausstellt, dass die erste Bearbeitung nicht ausreichend präzise war. Bei lebendem Gewebe wie beispielsweise humanem Augengewebe ist eine solche Vorgehensweise aus nachvollziehbaren Gründen nicht gangbar. Die Durchführung eines vorherigen Testbetriebslaufs der Scankomponenten kann Gewissheit darüber verschaffen, dass bei der anschließenden Laserbearbeitung tatsächlich Soll und Ist ausreichend genau beieinander liegen, oder/und sie kann Aufklärung über das Ausmaß etwaiger Abweichungen zwischen Soll und Ist verschaffen, so dass vor der Laserbearbeitung noch geeignete Korrekturmaßnahmen (z.B. Austausch zumindest eines Teils der Scankomponenten, Ermittlung von Korrekturfaktoren und Anpassung der Soll-Werte anhand der Korrekturfaktoren) veranlasst werden können.
Die Scanbahn definiert eine bestimmte Bewegungslinie, längs welcher der Strahlungsfokus während der Scanbewegung kontinuierlich bewegt wird, wobei der Hinweis auf den Strahlungsfokus nur der gedanklichen Klarstellung dient, da in dem Testbetriebslauf keine Laserstrahlung auf die Scankomponenten fällt und dementsprechend auch kein Strahlungsfokus vorhanden ist. Bei eingeschalteter Laserquelle würde freilich der dann vorhandene Strahlungsfokus der durch die Scanbahn vorgegebenen Bewegungslinie folgen.

Bevorzugt ist die Steueranordnung dazu eingerichtet, eine Erfassung der Ist-Fokusposition gemäß einer gegebenen Abtastrate in regelmäßigen zeitlichen Abständen zu bewirken. Die zeitliche Rate, mit welcher die Ist-Fokuspositionen erfasst werden (Abtastrate), kann unveränderbar vorgegeben sein oder sie kann vom Anwender wählbar sein. Bei gegebener Bahngeschwindigkeit, mit der sich der (gedachte) Strahlungsfokus längs der Scanbahn bewegt, kann die Steueranordnung auf Grundlage des gegebenen Bahnverlaufs, der Bahngeschwindigkeit und der Abtastrate zu jeder gemessenen Ist-Fokusposition die zugehörige Soll-Fokusposition ermitteln.
Typischweise ist der Bereich, innerhalb dessen der Strahlungsfokus verstellt werden kann, durch konstruktive, physikalische oder/und steuerungstechnische Vorgaben begrenzt. Der Anwender hat so ein vorgegebenes maximales Scanfeld zur Verfügung, das die äußeren Grenzen für die Scanbewegungen des Strahlungsfokus festlegt. Bei Ausführungsformen, die allein transversale Fokusbewegungen ermöglichen, ist das verfügbare Scanfeld demnach eine Transversalfläche. Bei Ausführungsformen dagegen, die transversale und longitudinale Fokusbewegungen ermöglichen, ist das verfügbare Scanfeld ein dreidimensionaler Raum. Transversal kann das verfügbare Scanfeld beispielsweise eine kreisförmige Außengrenze haben. Im dreidimensionalen Fall kann das verfügbare Scanfeld beispielsweise kreiszylindrische Form haben.
Zumindest bei einigen Anwendungen kann es sein, dass das verfügbare Scanfeld bis nahe an seine Grenzen heran für eine Applikation genutzt werden soll. Nicht nur, aber besonders dann ist es wichtig, Sicherheit zu haben, dass auch in den Randbereichen des verfügbaren Scanfelds die Scankomponenten präzise arbeiten und Soll/Ist-Abweichungen der Fokusposition, wenn überhaupt, nur in tolerablen Maßen auftreten. Zweckmäßigerweise definiert daher in der Erfindung das Test-Scanmuster mindestens eine Scanbahn für den Strahlungsfokus, welche an mindestens einer Stelle an der Grenze eines gegebenen maximalen Scanfelds verläuft. Die Scanbahn kann die Scanfeldgrenze beispielsweise einmal oder mehrmals punktuell berühren oder/und sie kann längs wenigstens eines Teils ihrer Bahnlänge (ggf. sogar auf ihrer gesamten Länge) entlang der Grenze verlaufen.

Das Test-Scanmuster kann mindestens eine in einer zur Strahlungsausbreitungsrichtung orthogonalen Transversalebene verlaufende Kreis-Scanbahn für den Strahlungsfokus definieren. Im Fall longitudinaler Scan-Fähigkeit der Scankomponenten kann das Test-Scanmuster sogar mehrere Kreis-Scanbahnen definieren, welche in verschiedenen Transversalebenen verlaufen, d.h. in Transversalebenen, welche longitudinal Abstand voneinander haben. Dabei kann beispielsweise eine erste Kreis-Scanbahn in einer ersten Transversalebene liegen, welche das verfügbare Scanfeld auf einer ersten longitudinalen Seite begrenzt. Eine zweite Kreis-Scanbahn kann in einer zweiten Transversalebene liegen, welche das verfügbare Scanfeld auf der gegenüberliegenden Longitudinalseite begrenzt. Sofern das Test-Scanmuster noch eine oder mehrere weitere Kreis-Scanbahnen definiert, können diese weiteren Kreis-Scanbahnen in transversalen Zwischenebenen liegen, welche zwischen den beiden endseitigen Transversalebenen der ersten und der zweiten Kreis-Scanbahn liegen.

Alternativ oder zusätzlich zu einer oder mehreren Kreis-Scanbahnen kann das Test-Scanmuster mindestens eine Schraubenbahn für den Strahlungsfokus mit einer längs der Strahlungsausbreitungsrichtung verlaufenden Schraubenachse definieren oder/und es kann das Test-Scanmuster mindestens eine mäandrierend in einer zur Strahlungsausbreitungsrichtung orthogonalen Transversalebene verlaufende Scanbahn definieren. Eine solche Mäander-Scanbahn kann beispielsweise von einem gedachten Kreis umgrenzt sein, an den sie an mehreren Stellen stößt. Das Anstoßen der Mäander-Scanbahn an den gedachten Begrenzungskreis kann zumindest teilweise punktuell sein; es kann auch linienhaft sein, wobei die Mäander-Scanbahn dann auf einem Teil ihrer Bahnlänge dem Begrenzungskreis folgt.

In einer bevorzugten Ausgestaltung weist die Mäander-Scanbahn eine Mehrzahl parallel zueinander in gegenseitigem Abstand verlaufender gerader Bahnstücke auf, die abwechselnd an gegenüberliegenden Enden jeweils paarweise miteinander verbunden sind. Auf diese Weise ist es möglich, das verfügbare Scanfeld nicht nur im Bereich seiner Ränder sondern auch in seinem Inneren großräumig auf Soll/Ist-Abweichungen zu untersuchen.

Um ein Maß für die Positionierpräzision der Scankomponenten zu gewinnen, kann die Steueranordnung dazu eingerichtet sein, Abweichungen zwischen den Soll-Fokuspositionen und den Ist-Fokuspositionen zu ermitteln und die ermittelten Abweichungen mit mindestens einer vorgegebenen Abweichungsschwelle zu vergleichen. Eine gewisse Anzahl von Positionierfehlern (d.h. Fällen, in denen die Soll/Ist-Abweichung eine zugeordnete Abweichungsschwelle übersteigt) kann tolerabel sein. In einer bevorzugten Ausgestaltung ist die Steueranordnung deshalb dazu eingerichtet, ab Erreichen einer vorgegebenen Anzahl die Abweichungsschwelle übersteigende Abweichungen die Ausgabe einer optisch oder/und akustisch wahrnehmbaren Warnmeldung zu bewirken. Zweckmäßig ist es, wenn zusätzlich zu der Warnmeldung die Steueranordnung in einen Sperrmodus eintritt, der eine Betätigung der Lasereinrichtung mit eingeschalteter Laserquelle unterbindet. Der Sperrmodus ist beispielsweise so ausgestaltet, dass ihn die Steueranordnung nur nach erfolgreicher Durchführung eines weiteren Testbetriebslaufs der Scankomponenten verlassen kann.

Allgemeiner ausgedrückt kann die Steueranordnung dazu eingerichtet sein, bei einer erfolglosen Durchführung des Testbetriebslaufs einen Betrieb der Lasereinrichtung mit eingeschalteter Laserquelle zu sperren.

Die Durchführung eines Testbetriebslaufs der Scankomponenten kann insbesondere nach einer längeren Standzeit der Lasereinrichtung sinnvoll sein, während der sie außer Betrieb war. Bevorzugt ist deshalb die Steueranordnung dazu eingerichtet, bei Hochfahren der Lasereinrichtung zur Herstellung ihrer Betriebsbereitschaft die Durchführung des Testbetriebslaufs programmgesteuert zu bewirken. Es versteht sich, dass andere auslösende Ereignisse definiert sein können, welche die Durchführung des Testbetriebslaufs bewirken.

Die Erfindung wird nachstehend anhand der beigefügten Zeichnungen näher erläutert. Es stellen dar:
- Fig. 1: in schematischer Blockdarstellung Elemente einer Lasereinrichtung nach einem Ausführungsbeispiel,
- Fig. 2: ein Beispiel einer Mäander-Scanbahn als Bestandteil eines von der Lasereinrichtung der Fig. 1 in einem Testbetriebslauf realisierbaren Test-Scanmusters,
- Fig. 3: eine Anordnung mehrerer in longitudinalem Abstand liegender transversaler Kreis-Scanbahnen als Bestandteil eines Test-Scanmusters und
- Fig. 4: ein Beispiel einer Schrauben-Scanbahn als Bestandteil eines Test-Scanmusters.

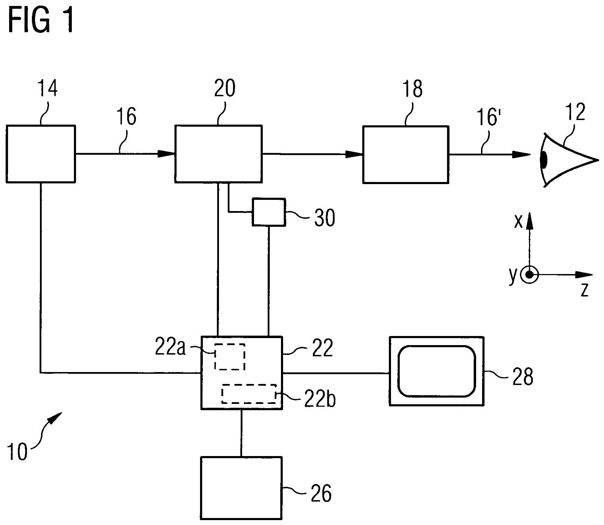

Die Lasereinrichtung der Fig. 1 - dort allgemein mit 10 bezeichnet - dient zur schneidenden Bearbeitung eines im Beispielfall als humanes Auge 12 gezeigten Objekts mittels ultrakurzpulsiger fokussierter Laserstrahlung. Ultrakurzpulsig meint hier Pulsdauern im Bereich von Femtosekunden bis höchstens einstelligen Pikosekunden. Der für die Schneidbearbeitung genutzte Effekt ist der sogenannte laserinduzierte optische Durchbruch, der zu einer Photodisruption innerhalb des bearbeiteten Materials (hier Augengewebe) führt. Durch Nebeneinandersetzen einer Vielzahl solcher Photodisruptionen können vielfältige Schnittfiguren in dem Auge 12 und dort vor allem in der Kornea erzeugt werden.

Die Lasereinrichtung 10 umfasst eine Fs-Laserquelle 14, die einen Laserstrahl 16 bereitstellt, welcher nach Durchlauf einer optischen Wegstrecke, längs der verschiedene Elemente zur Strahlführung und -formung angeordnet sind, als fokussierter Laserstrahl 16' auf das Auge 12 fällt. Die erwähnten Elemente zur Strahlführung und -formung umfassen eine beispielsweise von einem F-Theta-Objektiv gebildete Fokussieroptik 18 sowie hier schematisch durch einen einzigen Block angedeutete Scankomponenten 20. Es ist hervorzuheben, dass die Darstellung der Fokussieroptik 18 und der Scankomponenten 20 in Fig. 1 als getrennte Blöcke allein zum Zwecke der besseren Anschauung dient. Es ist ohne weiteres vorstellbar, dass ein Teil der für die Fokussierung des Laserstrahls 16 verantwortlichen optischen Bauteile auch Scanfunktionalität übernehmen kann. So ist es beispielsweise nicht ausgeschlossen, dass eine oder mehrere in der Fokussieroptik 18 enthaltene Linsen oder sogar die Fokussieroptik 18 als Ganzes zum Zwecke der longitudinalen Positionierung des Strahlfokus in Strahlausbreitungsrichtung verstellbar sind. Gleichwohl sind in einer bevorzugten Ausgestaltung die für das Scannen des Laserstrahls 16 dienenden optischen Bauteile von den zur Fokussierung des Laserstrahls 16 dienenden optischen Bauteilen separiert und infolgedessen außerhalb der Fokussieroptik 18 angeordnet.

Beispielsweise können die Scankomponenten zum transversalen Scannen des Laserstrahls 16 ein paar drehbar angeordneter Ablenkspiegel, deren Drehachsen senkrecht zueinander liegen, sowie in Zuordnung zu jedem der Ablenkspiegel einen individuell steuerbaren Galvanometerantrieb umfassen. Solche galvanometrisch betätigten Ablenkspiegel sind in der Fachwelt an sich bekannt; es bedarf daher an dieser Stelle keiner näheren Erläuterung hierzu. Zum longitudinalen Scannen des Strahlfokus können die Scankomponenten beispielsweise eine als Teil einer Strahlaufweitungsoptik (nicht näher dargestellt) vorgesehene Linse nutzen, die zum Zwecke der Divergenzvariation des Laserstrahls 16 in Strahlausbreitungsrichtung verstellbar angeordnet ist oder hinsichtlich ihrer Brechkraft justierbar ist. Ein zugehöriger Steller in Form eines Linearantriebs oder einer steuerbaren Spannungsquelle kann dann ebenfalls Teil der Scankomponenten 20 sein.

In einer Mindestausstattung der Lasereinrichtung 10 sind die Scankomponenten 20 jedenfalls zum transversalen Scannen des Laserstrahls 16 ausgelegt. In einer bevorzugten Ausgestaltung sind die Scankomponenten 20 zusätzlich zum longitudinalen Scannen eingerichtet. Es versteht sich im Übrigen, dass neben den erwähnten beispielhaften Ausgestaltungen der Scankomponenten andere Wirkprinzipien zum Einsatz kommen können, die ein transversales oder/und longitudinales Scannen ermöglichen, z. B. eine gesteuerte Strahlablenkung in einem elektrooptischen Kristall oder eine Divergenzbeeinflussung des Laserstrahls durch Verformung eines im Ausbreitungsweg des Laserstrahls 16 angeordneten optischen Spiegels.

Die Lasereinrichtung 10 enthält zudem eine prozessorbasierte Steueranordnung 22 zur Steuerung des Betriebs der Lasereinrichtung. Die Steueranordnung 22 ist programmgesteuert; in einer Speicheranordnung 26 ist das Steuerprogramm der Steueranordnung 22 abgelegt.

Wenngleich die Steueranordnung 22 in Fig. 1 durch einen einzelnen Block dargestellt ist, versteht es sich, dass ihre Steuerfunktionen auf verschiedene Steuermodule aufgeteilt sein können, die auf gesonderten Steuerplatinen in unterschiedliche Baumodule eingebaut sein können. So kann die Steueranordnung 22 beispielsweise ein gestrichelt bei 22a eingezeichnetes Scan-Steuermodul umfassen, das für die Steuerung der Scankomponenten 20 verantwortlich ist und zusammen mit diesen - oder zusammen mit zumindest einem Teil der Scankomponenten 20 - baulich in einen als gesondertes Bauteil vormontierten Scanner integriert ist. Die verbleibenden Steuerfunktionen der Steueranordnung 22 können beispielsweise in einem baulich außerhalb dieses Scanners gelegenen Zentral-Steuermodul 22b zusammengefasst sein, das unter anderem für die Synchronisation des Betriebs der Laserquelle 14 und des Betriebs der Scankomponenten 20 verantwortlich ist und dementsprechende Steuerbefehle an das Scan-Steuermodul 22a schicken kann, um einen Scanvorgang zu starten. Die konkreten Stelloperationen zur Einstellung der Scankomponenten 20 werden dann von dem Scan-Steuermodul 22a nach Maßgabe geeigneter Scandaten gesteuert, welche zuvor in das Scan-Steuermodul 22a geladen wurden und ein auszuführendes Scanmuster definieren.

Entsprechend der möglichen Aufteilung der Steueranordnung 22 in gesonderte Steuermodule können auch die Speicheranordnung 26 in gesonderte Speichermodule sowie das erwähnte Steuerprogramm in gesonderte Programmmodule aufgeteilt sein, die ihrerseits in verschiedenen Speichermodulen gespeichert sein können. Beispielsweise kann ein Speichermodul gemeinsam mit dem Scan-Steuermodul 22a in den erwähnten Scanner integriert sein und solche Programmteile speichern, die für die Steuerung der Scankomponenten 20 nötig sind. Ein oder mehrere weitere Speichermodule können dagegen dem Zentral-Steuermodul 22b zugeordnet sein und die übrigen Programmteile des Steuerprogramms speichern.

Mit der Steueranordnung 22 ist ferner eine Ausgabeeinheit 28 in Form eines Monitors verbunden, auf dem später noch zu erläuternde Testergebnisse ausgegeben werden können, die im Rahmen eines Testbetriebslaufs der Lasereinrichtung 10 gewonnen werden. Wenngleich in Fig. 1 nicht näher dargestellt, kann alternativ oder zusätzlich zu dem Monitor 28 ein Drucker an die Steueranordnung 22 angeschlossen sein, um die erwähnten Testergebnisse in gedruckter Form auszugeben.

In Fig. 1 ist zudem ein dreiachsiges Koordinatenkreuz eingezeichnet, das gemäß üblicher Notation eine zur Strahlungsausbreitungsrichtung des Laserstrahls 16 orthogonale x-y-Transversalebene aufspannt, während die z-Achse die Längsrichtung der Strahlausbreitung definiert.

Die Steueranordnung 22 ist dazu eingerichtet, bei abgeschalteter Laserquelle 14 einen Testbetriebslauf durchzuführen, bei dem die Scankomponenten 20 oder zumindest ein Teil derselben nach Maßgabe eines vorgegebenen Test-Scanmusters gesteuert werden. Dieser Trockenlauf, bei dem keine Laserstrahlung von der Laserquelle 14 abgegeben wird, soll eine Positionskontrolle ermöglichen, durch die sichergestellt werden soll, dass der gesamte Bereich, in dem der Strahlfokus in x-, y- und ggf. z-Richtung nominell eingestellt werden kann, tatsächlich angefahren werden kann. Insbesondere soll die Positionskontrolle eine Überprüfung etwaiger Soll/Ist-Abweichungen der Fokusposition in dem gesamten Scanbereich ermöglichen. Der maximale Scanbereich, der im vorliegenden Fall unter der Annahme sowohl transversaler als longitudinaler Scan-Fähigkeit ein dreidimensionaler Raum ist, wird hier auch als verfügbares Scanfeld bezeichnet.

Schematisch als einzelner Block angedeutete Messkomponenten (Messeinrichtung) 30 sind dazu vorgesehen, den tatsächlichen Einstellzustand wenigstens eines Teils der Scankomponenten 20 messtechnisch zu erfassen und entsprechende Messwerte an die Steueranordnung 22 zu liefern. Diese kann aus den gelieferten Messwerten Werte für die Ist-Position des Strahlfokus berechnen. Beispielsweise können zur Erfassung der Ist-Position eines in den Scankomponenten 20 enthaltenen drehbaren Ablenkspiegels die Messkomponenten 30 einen Positionsdetektor umfassen, wie er in EP 1 295 090 B1 gezeigt und beschrieben ist.

Die berechneten Ist-Fokuspositionen kann die Steueranordnung 22 in der Speicheranordnung 26 in Zuordnung zu zugehörigen Soll-Fokuspositionen abspeichern. Bei einer Schneidbearbeitung des Auges 12 sind die Soll-Fokuspositionen beispielsweise tabellarisch durch Angabe der jeweiligen x-, y- und z-Werte vorgegeben. Nach Abschluss der Schneidbearbeitung des Auges 12 kann die Steueranordnung 22 die zwischenzeitlich ermittelten Ist-Werte der Fokusposition mit den zugehörigen Soll-Werten vergleichen und entsprechende Informationen über den Monitor 28 oder einen angeschlossenen Drucker ausgeben. Der Operateur kann daraus die Qualität der Schneidbearbeitung und letztlich den Operationserfolg beurteilen.

Der zuvor erwähnte Testbetriebslauf ist vor allem dann (aber nicht nur) zweckmäßig, wenn während des Scanbetriebs der Lasereinrichtung 10 zwar mittels der Messkomponenten Messwerte für die Ist-Fokusposition aufgenommen und abgespeichert werden können, die Lasereinrichtung 10 aber nicht dazu eingerichtet ist, die Ist-Fokuspositionen während des Scanbetriebs im Hinblick auf Abweichungen von den zugehörigen Soll-Fokuspositionen auszuwerten und ggf. korrigierend einzugreifen, falls die Abweichungen zu groß sind. Solche Gegebenheiten können beispielsweise vorliegen, wenn die Soll- und Ist-Daten der Fokuspositionen während eines Scanvorgangs nicht von dem Scan-Steuermodul 22a zum Zentral-Steuermodul 22b übertragen werden können, sondern das Zentral-Steuermodul 22b erst nach Abschluss des Scanvorgangs Zugriff auf diese Daten bekommt. Der Testbetriebslauf ermöglicht so eine Vorabprüfung der Positionierqualität der Scankomponenten 20. Je nach Ergebnis des Testbetriebslaufs kann der Operateur oder ein automatisch arbeitendes Auswerteprogramm entscheiden, dass das verfügbare Scanfeld mit ausreichender Präzision angefahren werden kann, um eine geplante Augenbearbeitung durchzuführen, oder es können Maßnahmen wie ein Austausch zumindest eines Teils der Scankomponenten nötig sein, sollte sich der anfahrbare Scanbereich als unzureichend erweisen.

Der Testbetriebslauf kann durch die Steueranordnung 22 automatisch, d. h. ohne gesonderte Intervention eines Anwenders, gestartet werden, beispielsweise im Zuge des Systemstarts bei Einschalten eines nicht näher dargestellten Hauptschalters der Lasereinrichtung 10 oder/und zeitgesteuert nach Ablauf vorgegebener Zeitintervalle.

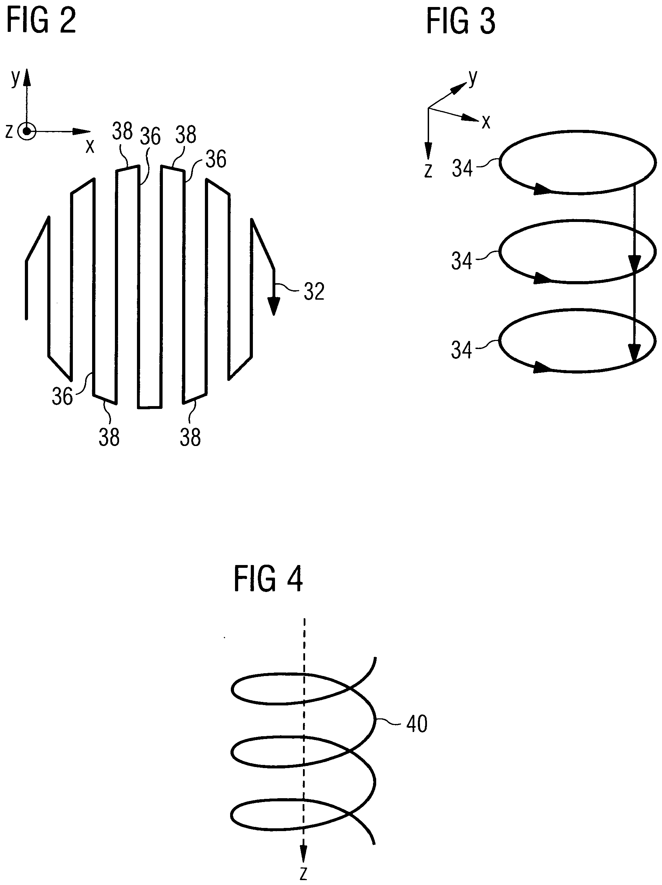

Zur Erläuterung des im Rahmen des Testbetriebslaufs ausgeführten Test-Scanmusters wird nun zusätzlich auf die Figuren 2 bis 4 verwiesen. Unter der Annahme eines kreiszylindrischen verfügbaren Scanfelds (d. h. eines Scanfelds, dessen transversale Außengrenze kreisförmig ist) kann das Test-Scanmuster beispielsweise eine in einer gegebenen Transversalebene abzufahrende Mäander-Scanbahn 32 oder/und mehrere (im gezeigten Beispielfall drei) in verschiedenen Transversalebenen abzufahrende Kreis-Scanbahnen 34 umfassen. Die in Fig. 2 gezeigte Mäander-Scanbahn 32 erstreckt sich innerhalb der Grenzen eines gedachten Umkreises (nicht näher dargestellt), der die transversale Außengrenze des verfügbaren Scanfelds markiert. Innerhalb dieses Umkreises ist die Mänder-Scanbahn 32 nach Art eines Schlangenlinienmusters mit einer Vielzahl parallel im Abstand nebeneinander verlaufender gerader Bahnstücke 36 ausgestaltet, wobei die Bahnstücke 36 abwechselnd an gegenüberliegenden Enden durch Verbindungsbahnstücke 38 miteinander verbunden sind. Sobald eines der geraden Bahnstücke 36 an den gedachten Umkreis stößt, schließt ein Verbindungsbahnstück 38 an, das entweder der Kreisbogenform des gedachten Umkreises folgt oder - wie in Fig. 2 dargestellt - einer Sehne des Umkreises entspricht. Mit einer solchen Mäander-Scanbahn 32 kann der in transversaler Richtung verfügbare Scanbereich sowohl an den Bereichsgrenzen als auch im Innern detailliert geprüft werden. Je nach gewünschter Dichte der Prüfstellen kann der gegenseitige Abstand der geraden Bahnstücke 36 enger oder weiter gewählt werden.

Die Kreis-Scanbahnen 34 der Fig. 3 dienen vor allem der Prüfung der Scanpräzision über die longitudinale Erstreckung (z-Länge) des verfügbaren Scanfelds. Die Kreis-Scanbahnen 34 können hierbei so dimensioniert sein, dass sie der transversalen Außengrenze des verfügbaren Scanfelds entsprechen. Es ist aber gleichwohl vorstellbar, für die Kreis-Scanbahnen 34 einen kleineren Durchmesser zu wählen, sodass sie radial innerhalb der transversalen Außengrenze des verfügbaren Scanfelds verlaufen.

In Longitudinalrichtung ist es zweckmäßig, wenn die beiden äußersten Kreis-Scanbahnen, in der Ansicht der Fig. 3 also die oberste und die unterste Kreis-Scanbahn, an den longitudinalen Bereichsgrenzen des Scanfelds liegen. Die mittlere der in Fig. 3 gezeigten Kreis-Scanbahnen 34 kann beispielsweise in der longitudinalen Mitte des verfügbaren Scanfelds liegen.

Die zeitlich Reihenfolge, in welcher die Mäander-Scanbahn 32 und die Kreis-Scanbahnen 34 abgefahren werden, ist an sich beliebig wählbar. Beispielsweise kann zunächst die Mäander-Scanbahn 32 abgefahren werden und anschließend die drei Kreis-Scanbahnen 34. Die umgekehrte Reihenfolge ist gleichermaßen vorstellbar. Auch kann zunächst eine Teilanzahl der Kreis-Scanbahnen 34 abgefahren werden, dann die Mäander-Scanbahn 32 eingefügt werden, bevor die verbleibende Restanzahl der Kreis-Scanbahnen 34 abgearbeitet wird.

Die Transversalebene, in der die Mäander-Scanbahn 32 liegt, kann der Transversalebene einer der Kreis-Scanbahnen 34 entsprechen. Sie kann alternativ in einer von den Kreis-Scanbahnen 34 verschiedenen Transversalebene ausgeführt werden. Für eine noch detailliertere Funktionskontrolle ist es sogar vorstellbar, die Mäander-Scanbahn 32 mehrmals in verschiedenen Transversalebenen auszuführen.

Eine weitere beispielhafte Test-Scanbahn, die im Testbetriebslauf an den Scankomponenten 20 eingestellt werden kann, ist in Figur 4 in Form einer Schrauben-Scanbahn 40 gezeigt. Die Schraubenachse der Schrauben-Scanbahn 40 verläuft vorzugsweise parallel zur z-Richtung, d.h. in Strahlungsausbreitungsrichtung. Zweckmäßigerweise verläuft die Schrauben-Scanbahn 40 an den Außengrenzen des verfügbaren Scanfelds. Im gezeigten Beispielfall umfasst sie drei Schraubenwindungen; es versteht sich jedoch, dass die Schrauben-Scanbahn sich aus einer größeren oder kleineren Anzahl von Windungen zusammensetzen kann.

Die in den Figuren 2 bis 4 gezeigten Test-Scanbahnen können im Rahmen der Erfindung einzeln oder in beliebiger Kombination in dem Test-Scanmuster enthalten sein.

Die Mäander-Scanbahn 32, die Kreis-Scanbahnen 34 und die Schrauben-Scanbahn 40 sind vorgegebene kontinuierliche Bahnen, zu deren Realisierung die Scankomponenten 20 entsprechend kontinuierlich justiert werden. Eine z.B. durch das Steuerprogramm der Steueranordnung 22 implementierte Zeitsteuerung veranlasst in regelmäßigen zeitlichen Abständen eine Erfassung des Ist-Einstellzustands zumindest eines Teils der Scankomponenten 20 durch die Messkomponenten 30 und damit eine Erfassung der Ist-Fokusposition. Diese Erfassung erfolgt, während die betreffende Scanbahn kontinuierlich abgefahren wird, d.h. während der für das Abfahren der Scanbahn benötigte Teil der Scankomponenten 20 kontinuierlich verstellt wird. Die Scankomponenten 20 werden also nicht angehalten, bevor eine Ist-Fokusposition erfasst wird. Nachdem der Testbetriebslauf ohne Laserstrahlung durchgeführt wird, handelt es sich bei der Ist-Fokusposition nur um eine gedachte Position, die dem Ist-Einstellzustand der Scankomponenten 20 entspricht, sich jedoch wegen des Fehlens eines Strahlfokus nicht in einer realen Fokusposition niederschlägt. Das zeitliche Abtastintervall der Ist-Fokusposition kann beispielsweise im Bereich von wenigen µs bis hin zu mehreren ms (beispielsweise etwa 10 ms) liegen. Die Anzahl der während eines Testbetriebslaufs gewonnenen Abtastwerte (d.h. die Anzahl der ermittelten Ist-Fokuspositionen) kann beispielsweise mehrere Hundert, mehrere Tausend oder sogar noch mehr betragen. Diese vergleichsweise große Anzahl von Abtastwerten ermöglicht eine zuverlässige Aussage über die korrekte Scannerfunktion im gesamten verfügbaren Scanfeld.

Zu jeder erfassten Ist-Fokusposition ermittelt die Steueranordnung 22 eine zugehörige Soll-Fokusposition. Diese kann sie aus dem bekannten Bahnverlauf der momentan ausgeführten Scanbahn (z. B. Mäander-Scanbahn 32 oder eine der Kreis-Scanbahnen 34), der Bahngeschwindigkeit, d. h. der Verstellgeschwindigkeit der Scankomponenten 20, sowie aus dem Zeitintervall ermitteln, mit dem die Ist-Fokusposition abgetastet wird. Die so ermittelten Soll-Fokuspositionen werden von der Steueranordnung 22 in Zuordnung zu den erfassten Ist-Fokuspositionen in der Speicheranordnung 26 abgespeichert.

Es ist darauf hinzuweisen, dass es je nach Scanbahn genügen kann, für die Soll-Fokuspositionen oder/und die Ist-Fokuspositionen nur eine oder zwei Koordinaten statt alle drei Koordinaten des x-, y- und z-Koordinatensystems zu berücksichtigen. Beispielsweise kann es für die Mäander-Scanbahn 32 genügen, die Soll-Fokuspositionen oder/und die Ist-Fokuspositionen nur durch die x- und y-Koordinatenwerte anzugeben. Was die Kreis-Scanbahnen 34 anbelangt, so kann es genügen, für die Soll-Fokuspositionen und die Ist-Fokuspositionen nur den z-Koordinatenwert und einen transversalen Koordinatenwert zu erfassen und zu speichern, beispielsweise den x-Koordinatenwert. Soweit die Lasereinrichtung 10 dies ermöglicht, kann es jedoch vorzuziehen sein, für sämtliche Soll-Fokuspositionen und Ist-Fokuspositionen alle drei Koordinatenwerte zu registrieren.

Nach Ausführung des Test-Scanmusters nimmt die Steueranordnung 22 eine Analyse der aufgenommenen Soll-Fokuspositionen und Ist-Fokuspositionen vor. Hierbei ermittelt sie Abweichungen zwischen den Soll-Fokuspositionen und den Ist-Fokuspositionen und vergleicht die gefundenen Abweichungen mit mindestens einer vorgegebenen Schwelle. Beispielsweise kann die Steueranordnung 22 die Abweichungen zwischen den Soll-Fokuspositionen und den zugehörigen Ist-Fokuspositionen getrennt für jede Koordinatenachse einzeln ermitteln und mit einer zugeordneten Abweichungsschwelle vergleichen. Die anzuwendende Abweichungsschwelle kann für die Koordinatenachsen gleich sein oder für unterschiedliche Koordinatenachsen unterschiedlich sein. Gewünschtenfalls kann die Steueranordnung 22 zusätzlich eine Gesamtabweichung aus den längs der einzelnen Koordinatenachsen gefundenen Abweichungen ermitteln, etwa nach Art des euklidischen Abstands. Auch die so gefundene Gesamtabweichung kann sie mit einer zugeordneten Abweichungsschwelle vergleichen. Es versteht sich, dass jede Abweichungsschwelle entweder fest vorgegeben sein kann oder vom Anwender über eine nicht näher dargestellte, mit der Steueranordnung 22 verbundene Eingabeeinheit wählbar sein kann.

Teil der Auswertung der Steueranordnung 22 kann eine Plausibilitätsprüfung sein. Ist-Fokuspositionen, die übermäßig von ihrer zugeordneten Soll-Fokusposition abweichen, können von der Steueranordnung 22 als unplausibel verworfen werden und bei der weiteren Auswertung unberücksichtigt gelassen werden.

Die Steueranordnung 22 zählt diejenigen Fälle, bei denen eine Soll/Ist-Abweichung eine zugehörige Abweichungsschwelle übersteigt. Sobald die Anzahl der eine gegebene Abweichungstoleranz überschreitenden Fälle eine bestimmte vorgegebene Grenze erreicht, veranlasst die Steueranordnung 22 die Ausgabe eines Warnhinweises auf dem Monitor 28. Der Warnhinweis teilt dem Anwender mit, dass die Scanpräzision nicht ausreichend ist. Als weitere Reaktion auf das Erreichen der Grenze für die Anzahl zulässiger Fehler kann die Steueranordnung 22 in einen Sperrmodus eintreten, der einen Betrieb der Lasereinrichtung 10 mit eingeschalteter Laserquelle 14 unterbindet. Erst nach erfolgreicher Durchführung eines weiteren Testbetriebslaufs, bei dem die Anzahl der über der Abweichungsschwelle liegenden Soll/Ist-Abweichungen unter der zulässigen Grenze geblieben ist, wird der Sperrmodus wieder aufgehoben und der Betrieb der Lasereinrichtung 10 mit eingeschalteter Laserquelle 14 ermöglicht.

Alternativ oder zusätzlich kann die Steueranordnung 22 dazu eingerichtet sein, die ermittelten Soll/Ist-Abweichungen nicht näher auszuwerten und sie ggf. zusammen mit den Ist-Fokuspositionen oder/und den Soll-Fokuspositionen auf dem Monitor 28 oder in anderer Form auszugeben. In diesem Fall bleibt die Bewertung des Testergebnisses dem Anwender überlassen.

Die soweit offenbarte Methodik erlaubt eine Prüfung, ob das dynamische Verhalten der Scankomponenten 20 (allgemein: des Scansystems) den spezifizierten Anforderungen entspricht und ob der verfügbare Scanraum in seiner Gesamtheit genutzt, d.h. angefahren, werden kann. Hierzu definiert das Test-Scanmuster vorzugsweise eine oder mehrere Scanbahnen, welche derart in dem verfügbaren maximalen Scanraum liegen, dass durch Abfahren dieser Scanbahnen sichergestellt werden kann, dass das Scansystem den gesamten möglichen Scanbereich sauber anfahren kann und an keiner Stelle Probleme auftreten. Das Abfahren der Scanbahnen stellt eine kontinuierliche Bewegung dar, das Scansystem hält also an den Abtaststellen nicht zum örtlichen Einschwingen an. Diese zeitlich getriggerte Positionsmessung bei kontinuierlicher Scanbewegung kann zum Auftreten von Schleppfehlern führen, die jedoch als leicht verminderte Messgenauigkeit in Kauf genommen werden können, da trotz solcher Schleppfehler der angestrebte Nachweis der vollen Ausnutzbarkeit des verfügbaren maximalen Scanraums erbracht werden kann.

## Patentansprüche

1. Lasereinrichtung, insbesondere für die ophthalmologische Laserchirurgie, umfassend
- eine Laserquelle (14) zur Bereitstellung von Laserstrahlung,
- steuerbare Scankomponenten (20) zur Einstellung einer Fokusposition der Laserstrahlung,
- Messkomponenten (30) zur Erfassung von Informationen, welche für eine Ist-Position des Strahlungsfokus repräsentativ sind,
- eine die Laserquelle (14) und die Scankomponenten (20) steuernde Steueranordnung (22), welche dazu eingerichtet ist, die Durchführung eines Testbetriebslaufs mindestens eines Teils der Scankomponenten bei abgeschalteter Laserquelle nach Maßgabe eines vorgegebenen Test-Scanmusters zu bewirken, wobei das Test-Scanmuster mindestens eine Scanbahn (32, 34) für den Strahlungsfokus definiert und die Steueranordnung (22) dazu eingerichtet ist, während einer Scanbewegung entlang der Scanbahn mehrmals hintereinander eine Erfassung der Ist-Fokusposition ohne Anhalten der Scanbewegung zu bewirken und in Zuordnung zu jeder erfassten Ist-Fokusposition eine Soll-Fokusposition zu ermitteln, **dadurch gekennzeichnet, dass** das Test-Scanmuster mindestens eine Scanbahn (32, 34) für den Strahlungsfokus definiert, welche an mindestens einer Stelle an der Grenze eines gegebenen maximalen Scanfelds verläuft, das durch konstruktive Vorgaben begrenzt ist.

2. Lasereinrichtung nach Anspruch 1, wobei die Steueranordnung (22) dazu eingerichtet ist, eine Erfassung der Ist-Fokusposition gemäß einer gegebenen Abtastrate in regelmäßigen zeitlichen Abständen zu bewirken.

3. Lasereinrichtung nach einem der vorhergehenden Ansprüche, wobei das Test-Scanmuster mindestens eine in einer zur Strahlungsausbreitungsrichtung orthogonalen Transversalebene verlaufende Kreis-Scanbahn (34) für den Strahlungsfokus definiert.

4. Lasereinrichtung nach Anspruch 3, wobei das Test-Scanmuster mehrere Kreis-Scanbahnen (34) definiert, welche in verschiedenen Transversalebenen verlaufen.

5. Lasereinrichtung nach einem der vorhergehenden Ansprüche, wobei das Test-Scanmuster mindestens eine mäandrierend in einer zur Strahlungsausbreitungsrichtung orthogonalen Transversalebene verlaufende Scanbahn (32) definiert.

6. Lasereinrichtung nach Anspruch 5, wobei die Mäander-Scanbahn (32) von einem gedachten Kreis umgrenzt ist, an den sie an mehreren Stellen stößt.

7. Lasereinrichtung nach einem der vorhergehenden Ansprüche, wobei das Test-Scanmuster mindestens eine Schraubenbahn für den Strahlungsfokus mit einer längs der Strahlungsausbreitungsrichtung verlaufenden Schraubenachse definiert.

8. Lasereinrichtung nach einem der vorhergehenden Ansprüche, wobei die Steueranordnung (22) dazu eingerichtet ist, Abweichungen zwischen den Ist-Fokuspositionen und zugehörigen Soll-Fokuspositionen zu ermitteln und die ermittelten Abweichungen mit mindestens einer vorgegebenen Abweichungsschwelle zu vergleichen.

9. Lasereinrichtung nach Anspruch 8, wobei die Steueranordnung (22) dazu eingerichtet ist, ab Erreichen einer vorgegebenen Anzahl die Abweichungsschwelle übersteigender Abweichungen die Ausgabe einer optisch oder/und akustisch wahrnehmbaren Warnmeldung zu bewirken.

10. Lasereinrichtung nach einem der vorhergehenden Ansprüche, wobei die Steueranordnung (22) dazu eingerichtet ist, bei einer erfolglosen Durchführung des Testbetriebslaufs einen Betrieb der Lasereinrichtung mit eingeschalteter Laserquelle zu sperren.

11. Lasereinrichtung nach einem der vorhergehenden Ansprüche, wobei die Steueranordnung (22) dazu eingerichtet ist, bei Hochfahren der Lasereinrichtung zur Herstellung ihrer Betriebsbereitschaft die Durchführung des Testbetriebslaufs programmgesteuert zu bewirken.

## Claims

1. Laser device, in particular for ophthalmological laser surgery, comprising
- a laser source (14) for providing laser radiation,
- controllable scanning components (20) for setting a focal position of the laser radiation,
- measuring components (30) for capturing information which is representative of an actual position of the radiation focus,
- a control arrangement (22) which controls the laser source (14) and the scanning components (20) and which is configured to effectuate the carrying out of a test mode run of at least some of the scanning components in accordance with a predetermined test scanning pattern when the laser source is switched off, wherein the test scanning pattern defines at least one scanning path (32, 34) for the radiation focus and the control arrangement (22) is configured to effectuate the capture of the actual focal position a number of times in succession during a scanning movement along the scanning path, without an interruption of the scanning movement, and ascertain an intended focal position assigned to each captured actual focal position,
**characterized in that** the test scanning pattern defines at least one scanning path (32, 34) for the radiation focus which scanning path, at at least one point, extends along the boundary of a given maximum scanning field which is restricted by structural specifications.

2. Laser device according to Claim 1, wherein the control arrangement (22) is configured to effectuate the capture of the actual focal position at regular temporal intervals as per a given scanning rate.

3. Laser device according to any one of the preceding claims, wherein the test scanning pattern defines at least one circular scanning path (34) for the radiation focus that extends in a transversal plane that is orthogonal to the radiation propagation direction.

4. Laser device according to Claim 3, wherein the test scanning pattern defines a plurality of circular scanning paths (34) which extend in different transversal planes.

5. Laser device according to any one of the preceding claims, wherein the test scanning pattern defines at least one scanning path (32) that extends in meandering fashion in a transversal plane that is orthogonal to the radiation propagation direction.

6. Laser device according to Claim 5, wherein the meandering scanning path (32) is bounded by an imaginary circle, which it touches at a number of points.

7. Laser device according to any one of the preceding claims, wherein the test scanning pattern defines at least one helical path for the radiation focus, with a helical axis that extends along the radiation propagation direction.

8. Laser device according to any one of the preceding claims, wherein the control arrangement (22) is configured to ascertain deviations between the actual focal positions and associated intended focal positions and compare the ascertained deviations with at least one predetermined deviation threshold.

9. Laser device according to Claim 8, wherein the control arrangement (22) is configured to effectuate the output of an optically and/or acoustically perceivable warning message once a predetermined number of deviations that exceed the deviation threshold has been reached.

10. Laser device according to any one of the preceding claims, wherein the control arrangement (22) is configured to block an operation of the laser device with a switched-on laser source if the test mode run is carried out unsuccessfully.

11. Laser device according to any one of the preceding claims, wherein the control arrangement (22) is configured to effectuate the carrying out of the test mode operation in a program-controlled manner when starting up the laser device for the purposes of achieving operational readiness of the latter.

## Revendications

1. Dispositif laser, en particulier pour la chirurgie ophtalmique au laser, comprenant
- une source laser (14) destinée à fournir un rayonnement laser,
- des composants de balayage (20) commandés destinés à régler une position de focalisation du rayonnement laser,
- des composants de mesure (30) destinés à détecter des informations qui sont représentatives d'une position effective de la focalisation du rayonnement,
- un agencement de commande (22) commandant la source laser (14) et les composants de balayage (20), qui est agencé pour provoquer la réalisation d'un cycle d'essai d'au moins une partie des composants de balayage alors que la source laser est hors circuit conformément à un motif de balayage d'essai prédéfini, le motif de balayage d'essai définissant au moins une trajectoire de balayage (32, 34) pour la focalisation du rayonnement, et l'agencement de commande (22) étant agencé pour, pendant un mouvement de balayage le long de la trajectoire de balayage, provoquer plusieurs fois successivement une détection de la position effective de focalisation sans arrêt du mouvement de balayage et pour déterminer une position de consigne de focalisation en association avec chaque position effective de focalisation détectée, **caractérisé en ce que** le motif de balayage d'essai définit au moins une trajectoire de balayage (32, 34) pour la focalisation du rayonnement qui s'étend en au moins un endroit à la limite d'un champ de balayage maximal donné qui est limité par des spécifications de construction.

2. Dispositif laser selon la revendication 1, l'agencement de commande (22) étant agencé pour provoquer une détection de la position effective de focalisation conformément à une fréquence de balayage donnée à intervalles de temps réguliers.

3. Dispositif laser selon l'une des revendications précédentes, le motif de balayage d'essai définissant pour la focalisation du rayonnement au moins une trajectoire de balayage circulaire (34) s'étendant dans un plan transversal qui est orthogonal à la direction de propagation du rayonnement.

4. Dispositif laser selon la revendication 3, le motif de balayage d'essai définissant plusieurs trajectoires de balayage circulaire (34) qui s'étendent dans différents plans transversaux.

5. Dispositif laser selon l'une des revendications précédentes, le motif de balayage d'essai définissant au moins une trajectoire de balayage (32) s'étendant de façon méandreuse dans un plan transversal qui est orthogonal à la direction de propagation du rayonnement.

6. Dispositif laser selon la revendication 5, la trajectoire de balayage (32) méandreuse étant entourée d'un cercle imaginaire contre lequel elle bute en plusieurs endroits.

7. Dispositif laser selon l'une des revendications précédentes, le motif de balayage d'essai définissant au moins une trajectoire hélicoïdale pour la focalisation du rayonnement avec un axe hélicoïdal s'étendant le long de la direction de propagation du rayonnement.

8. Dispositif laser selon l'une des revendications précédentes, l'agencement de commande (22) étant agencé pour déterminer des divergences entre les positions effectives de focalisation et des positions de consigne de focalisation correspondantes et pour comparer les divergences déterminées avec au moins un seuil de divergence prédéfini.

9. Dispositif laser selon la revendication 8, l'agencement de commande (22) étant agencé pour, une fois atteint un nombre prédéfini de divergences dépassant le seuil de divergence, provoquer la délivrance d'un avertissement pouvant être perçu optiquement ou visuellement.

10. Dispositif laser selon l'une des revendications précédentes, l'agencement de commande (22) étant agencé pour, lors d'un échec de la réalisation du cycle d'essai, bloquer un fonctionnement du dispositif laser avec la source laser mise en circuit.

11. Dispositif laser selon l'une des revendications précédentes, l'agencement de commande (22) étant agencé pour, lors du démarrage du dispositif laser en vue de l'établissement de son état opérationnel, provoquer la réalisation du cycle d'essai sous la commande d'un programme.
